# EUROPEAN PATENT APPLICATION

(11) **EP 1 972 361 A1**
(43) Date of publication of application: **24.09.2008**
(21) Application number: 07005747.6
(22) Date of filing: 21.03.2007
(51) Int. Cl.: A61Q 19/10, A61Q 11/00, A61K 8/11

(54) **Mild cleansing compositions with an encapsultated dye**

(71) Applicant: Cognis IP Management GmbH, 40589 Düsseldorf (DE)
(72) Inventor: Viladot Petit, Josep-Lluis, 08018 Barcelona (ES); De Moragas Maria, 08310 Argentona (Barcelona) (ES); Gomez, Yolanda, 08170 Montornés del Vallés (ES); Asensio, Juan-Antonio, 08820, El Prat de Llobregat (ES); Martinez, Silvia, 08173 St. Cugat del Vallés (ES); Sanchez Agustin, 08921 Santa Coloma De Gramenet (Barcelona) (ES)
(74) Representative: Fabry, Bernd

(57) **Abstract**

A mild cleaning composition comprising an anionic, non-ionic and / or amphoteric surfactant in combination with a first dye (or dyes) and encapsulated second dye(s) having a different colour from the first. The composition is intended for use in personal care compositions, manual dish washing compositions, light duty detergents and toothpastes.

## Description

### Object of the invention

The present invention relates to mild cleansing compositions, more particularly to personal care compositions, manual dish wash compositions or light duty detergents comprising two dyes of different colour, one encapsulated and the other in the continuous phase of the composition.

### State of the art

Cleansing compositions, in particular personal care compositions represent an important part of the daily hygienic regimen for a large group of both women and men throughout the world. Particularly hand cleansing products have traditionally been marketed in a variety of forms such as bar soaps, creams, lotions, pastes and gels. The amount of time needed to clean the skin or a surface has been researched extensively. The Association for Professionals in Infection Control and Epidemiology (APIC) *Guideline for Hand Washing and Hand Antisepsis in Health-Care Settings* (1995), for example, recommends a wash time of 10 to 15 seconds with soap or detergent for routine hand washing for general purposes. The APIC recommends an antimicrobial soap or detergent or alcohol-based rub wash of 10-15 seconds to remove or destroy transient micro-organisms in, for example, nursing and food preparation applications. The APIC further recommends an antimicrobial soap or detergent used with brushing for at least 120 seconds for surgical applications. The US Centres for Disease Control and Prevention (CDC) recommends up to 5 minutes of hand cleaning for surgical applications. Clearly, the length of time spent washing the hands or brushing the teeth can have a great effect on the eradication of microbes. Thus there is a need for a cleaning formulation that will enable the user to judge how long he has washed his hands or brushed his teeth in order to comply with the guidelines.

Proper personal care habits are also important for children. Children in particular need guidance in determining the appropriate amount of time in which hand washing, showering or the brushing of the teeth should be performed. This guidance is generally given by parents or other caregivers and, while important, is not omnipresent. In addition to parental guidance, various other mechanisms have been used to encourage children to increase their cleansing times. Soaps have been formulated as foams, for example, to increase the enjoyment children find in hand washing and thus to increase the amount of time children spend by washing. Also luminescent dyes have been added to shampoo formulations which irradiate light when the kids take their shower in the dark.

The problem explained above, however is not limited to personal care, but is also associated with manual dish washing or light duty detergents. In both areas it is desirous to have a optical sign which indicates that the cleansing has reached a sufficient degree.

Nevertheless, a huge state of the art exists which refers to optical indicators in cleansing compositions. For example FR 2,805,162 A1 (J.C. Casella) discloses a process for the visual control of hand washing by change of colour. This can be achieved by decomposition for example of microcapsules releasing a dye, like methylene blue

WO 04/041293 A1 (Venture Management Alliance) refers to cosmetic composition, comprising (a) a non-aqueous carrier, (b) a plurality of non-aqueous carrier stable capsules, and (c) at least on sensorial perceivable material. The invention particularly refers to personal care compositions for washing the hands comprising encapsulated dyes. A sufficient degree of cleaning is shown by the change of colour.

US 2005/0049157 A1 (Kimberley-Clark) suggests a colour change composition that remains stable in a single phase and that contains an indicator that produces an observable colour change after a period of time to show that sufficient cleaning has been done or to indicate the thoroughness of the cleaning. The proposed compositions comprise redox dyes which change their colour when brought into contact with oxygen. This solution, however, is not satisfying since the compositions must be kept strictly away from air contact in order to avoid undesired colour development.

US 2005/ 0112152 A1 discloses encapsulated fragrance materials. The encapsulated fragrance and solvent materials can be further coated with a second coating, preferably a cationic coating, and be added to personal care compositions.

The problem underlying the present invention has therefore been the development of cleansing compositions, more particularly, of personal care compositions like shower gels or hand wash soaps, manual dish wash compositions or light duty detergents, which simultaneously
o exhibit strong cleansing power combined with
o high dermatological mildness and very low irritation potential,
o which provide an optical signal to the user after a sufficient degree of cleansing has been reached and
o allows the stable incorporation of encapsulated actives even over longer storage times and higher storage temperatures.

### Description of the invention

The present invention mild cleansing compositions comprising
(a) Anionic, non-ionic and/or amphoteric surfactants
(b) Dyes and
(c) Encapsulated dyes, showing a colour different from the dyes of component (b).

Surprisingly it has been found that the addition of encapsulated dyes to cleansing compositions solves the problem outlined above. In the presence of mild surfactants the capsules are stable enough to survive some mechanical exposure, however break after a certain time releasing the dye which indicates that a sufficient degree of cleansing has been achieved by changing the colour of the composition. A special advantage of the composition according to the invention is that it can stay in contact with air without any undesired development of colour; this is especially the case when the capsules are obtained from the reaction of anionic and cationic polymers. The invention includes also the teaching according to which certain anionic, non-ionic or amphoteric surfactants exhibit not only a high cleansing power combined with extreme mildness, but also allow the incorporation of capsules without dissolving the shell during storage and releasing the encapsulated dyes too early.

In a preferred embodiment, cleansing compositions according to the present invention represent personal care compositions, manual dish washing compositions or light duty detergents, more particularly liquid soaps or hand washing pastes. It also preferred that the compositions show a viscosity according to Brookfield (RVT, spindle 3, 10 rpm) of 300 to 30,000, more preferably 1,000 to 5,000, and most preferably 2,000 to 3,000 mPas.

### Mild surfactants

From literature a huge number of surfactants are known which are useful for very different applications. Although it is very common that surfactants over all show a sufficient cleansing performance, significant differences exist when it comes to a comparison of their dermatological behaviour. Often, those products showing the highest cleansing power are also those which cause skin irritation. It is therefore desirous to identify those surfactants which combine good performance and extreme mildness in order to solve the underlying problem. Also these surfactants should allow using very different types of micro-capsules without dissolving their shells and liberating the encapsulated dyes too early. Among the surfactants meeting these requirements the following preferred examples have been chosen to illustrate the invention without limiting it. The selection has been done taking into account the very advantageous behaviour of the surfactants as explained above.

### N-acylamino acids

Basically, the N-acylamino acids which form component (a1) may be derived from any α-amino acids which can be acylated with fatty acid halides to form N-acylamino acids. Preferred amino acids are glutamic acid, sarcosine, aspartic acid, alanine, valine, leucine, isoleucine, proline, hydroxyproline, lysine, glycine, serine, cystein, cystine, threonine, histidine and salts thereof and, more particularly, glutamic acid, sarcosine, aspartic acid, glycine, lysine and salts thereof. The amino acids may be used in optically pure form or as racemic mixtures. The amino acid components of the N-acylamino acids are preferably derived from glutamic acid and/or aspartic acid, i.e. N-acyl glutamates and N-acyl aspartates are preferably used.

In addition, the acyl groups of the N-acylamino acids may be derived from fatty acids corresponding to formula (**I**):

**R¹CO-OH** **(I)**

in which R¹ is a linear or branched acyl group containing 6 to 22 carbon atoms and 0 and/or 1 to 3 double bonds. Typical examples are acyl groups derived from caproic acid, caprylic acid, capric acid, lauric acid, mrystic acid, palmitic aid, stearic acid, isostearic acid, oleic acid, elaidic acid, linoleic acid, linolenic acid, gadoleic acid, arachidonic acid, behenic acid and erucic acid and technical mixtures thereof. The N-acylamino acids are preferably derived from technical C₁₂₋₁₈ coconut oil fatty acids. The N-acylamino acids may be present in acidic form, but are generally used in the form of their salts, preferably alkali metal or ammonium salts.

The sodium and triethanolamine salts are particularly preferred. Overall, N-cocoyl glutamate is the preferred N-acylamino acid. Products can be found in the market for example under the trademark Plantapon^{®} ACG (Cognis Deutschland GmbH).

### Alk(en)yl oligoglycoside carboxylates

The alk(en)yl oligoglycoside carboxylates which can be used in the compositions according to the invention as component (**a2**) correspond to formula **(II)** :

**R²O[G]ₚO[(CH₂)ₘCOO⁻X⁺]ₙ** **(II)**

wherein R² is an alkyl or alkenyl radical having from 6 to 22 carbon atoms, G is a sugar unit having 5 or 6 carbon atoms, p is a number from 1 to 10, m and n are numbers from 1 to 5, and X is alkali, alkaline earth, ammonium, alkylammonium, alkanolammonium or glucammonium. The products are obtainable according to the methods known from the art, for example by reaction of alk(en)yl oligoglycosides with halogen carboxylic acids or their salts in alkaline medium an in the presence of solvents. The alkyl or alkenyl oligoglycosides carboxylates may be derived from aldoses or ketoses containing 5 or 6 carbon atoms, preferably glucose. Accordingly, the preferred alkyl and/or alkenyl oligoglycoside carboxylates are alkyl or alkenyl oligo**glucoside** carboxylates. The index p in general formula (**II**) indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside moiety is an analytically determined calculated quantity which is mostly a broken number. Alk(en)yl oligoglycoside carboxylates having an average degree of oligomerisation p of 1.1 to 3.0 are preferably used. Alk(en)yl oligoglycoside carboxylates having a degree of oligomerisation below 1.7 and, more particularly, between 1.2 and 1.4 are preferred from the applicational point of view. The alkyl or alkenyl radical R² may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides carboxylates based on hydrogenated C₈-C₁₆ coconut oil alcohol having a DP of 1 to 3 are preferred. Furtheron, the alk(en)yl oligoglycoside carboxylates may be derived from carboxylic acids, their salts or esters, in which the acyl moiety comprises 1 to 5, preferably 2 to 4 and more preferably 1 or 2 carbon atoms, while the number of acyl groups in the alk(en)yl oligoglycoside carboxylate may be 1 to 5 and preferably 1 to 3. Moreover, X stands preferably for potassium, ammonium, triethanolammonium and most preferably for sodium. The carboxylic acids which can be used for preparing the alk(en)yl oligoglycoside carboxylates usually comprise 1 to 4 carbon atoms; preferably acetic acid, its esters or its salts, particularly its sodium or potassium salt are used. In a preferred embodiment of the present invention alk(en)yl oligoglycoside carboxylates are used which are obtained by reaction of an aqueous solution of an alk(en)yl oligoglycoside, having e.g. 20 to 70 % b.w. solids matter, under nitrogen and in presence of an alkaline catalyst, e.g. alkali hydroxide or alkali carbonate, at a temperature of from 50 to 100 °C with ω-halogen carboxylic acid, its ester or salt, like for example potassium or sodium monocloroacetate in a molar ratio of from 1 : 0.5 to 1 : 5, preferably 1 : 1 to 1 : 3. The molar ratio of alkali: halogen carboxylic acid, its esters or salts is usually adjusted to 1 : 0.5 to 1 : 1.5 and preferably 1 : 1.1. The preparation of the medium chain C_{12/14} alkyl oligoglycoside carboxylates usually takes place in the absence of organic solvents, while the long chain C_{16/18} alkyl oligoglycoside carboxylates are prepared in the presence of long chain fatty alcohols or 1,2-propylene glycol. These products are obtainable in the market for example under the trademark Plantapon^{®} LGC (Cognis Deutschland GmbH & Co. KG).

### Alk(en)yl polyalkylene glycol ether citrates

Alk(en)yl polyalkylene glycol ether citrates, which form component (a3), represent mixtures of mono, di and triesters of citric acid and alkoxylated alcohols following formula (**III**) 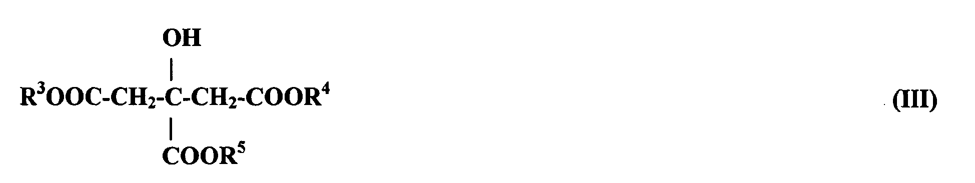 in which R³, R⁴, and R⁵ independently stand for hydrogen or a radical (IV)

**R⁶(OCH₂CHR⁷)ₙ** **(IV)**

in which R⁶ is a linear or branched alkyl and/or alkenyl group containing 6 to 22 carbon atoms, R⁷is hydrogen or methyl, and n is a number of 1 to 20, under the condition that at least R³, R⁴, or R⁵ is different from hydrogen. Typical examples for the alcohol part of the esters are the addition products of on average 1 to 20 and, more particularly, 5 to 10 moles of ethylene oxide and/or propylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof. The ethers may have both a conventional homologue distribution and a narrow homologue distribution. It is particularly preferred to use alkyl polyalkylene glycol ethers based on addition products of, on average, 5 to 10, and more particularly of about 7 moles of ethylene oxide with technical C₁₂-C₁₈ , preferably C₁₂-C₁₄ fatty alcohol fractions. These products are obtainable in the market for example under the trademark Plantapon^{®} LC7 (Cognis Deutschland GmbH & Co. KG).

### Alk(en)yl polyalkylene glycol ether sulfates

It is known that alk(en)yl polyalkylene glycol ether sulfates ("ether sulfates") which form component (a4) are anionic surfactants which are industrially produced by the sulfation of fatty alcohol or oxoalcohol polyglycol ethers with SO₃ or chlorosulfonic acid (CSA) and subsequent neutralization. Alk(en)yl ether sulfates suitable for the purposes of the invention correspond to formula (V):

**R⁸(OCH₂CHR⁹)ₙOSO₃X** **(I)**

in which R⁸ is a linear or branched alkyl and/or alkenyl group containing 6 to 22, more particularly 12 to 18 carbon atoms, R⁹ is hydrogen or methyl, n is a number of 1 to 5 and X is alkali, alkaline earth, ammonium, alkylammonium, alkanolammonium or glucammonium. Typical examples are the sulfates of addition products of on average 1 to 5 and, more particularly, 2 to 3 moles of ethylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof in the form of their alkyl, preferably monoisopropanolammonium salts. The ether sulfates may have both a conventional homologue distribution and a narrow homologue distribution. It is particularly preferred to use ether sulfates based on addition products of, on average, 2 to 3 moles of ethylene oxide with technical C₁₂-C₁₈ , preferably C₁₂-C₁₄ fatty alcohol fractions preferably in the form of their sodium, magnesium, ammonium or monoisopropanolammonium salts. These products are obtainable in the market for example under the trademark Texapon^{®} NSO (Cognis Deutschland GmbH & Co. KG).

### Alk(en)yl oligoglycosides

The alkyl or alkenyl oligoglycosides which can be used in the compositions according to the invention as component (a4) may be derived from aldoses or ketoses containing 5 or 6 carbon atoms, preferably glucose. Accordingly, the preferred alkyl and/or alkenyl oligoglycosides are alkyl or alkenyl oligo**glucosides**. These materials are also known generically as "alkyl polyglycosides" (APG). The alk(en)yl oligoglycosides according to the invention correspond to formula (VI) :

**R¹⁰O[G]ₚ** **(VI)**

wherein R¹⁰ is an alkyl or alkenyl radical having from 6 to 22 carbon atoms, G is a sugar unit having 5 or 6 carbon atoms and p is a number from 1 to 10. The index p in general formula (**VI**) indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside is an analytically determined calculated quantity which is mostly a broken number. Alk(en)yl oligoglycosides having an average degree of oligomerisation p of 1.1 to 3.0 are preferably used. Alk(en)yl oligoglycosides having a degree of oligomerisation below 1.7 and, more particularly, between 1.2 and 1.4 are preferred from the applicational point of view. The alkyl or alkenyl radical R¹⁰ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred. These products are obtainable in the market for example under the trademark Plantaren^{®} APG or Glucopon^{®} APG (Cognis Deutschland GmbH & Co. KG).

### Alkyl betaines and Alkylamido betaines

Alkyl betaines, forming component (a6) represent known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid for example, is also possible. Examples of suitable betaines are the carboxyalkylation products of secondary and, in particular, tertiary amines which correspond to formula **(VII):** 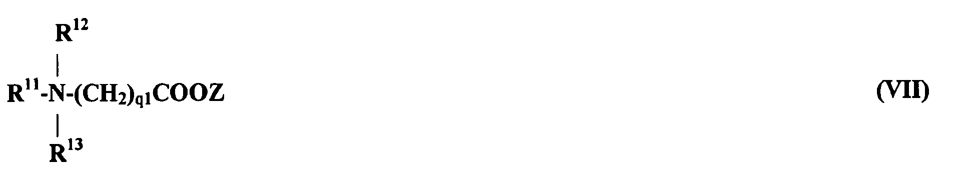 where R¹¹ is a an alkyl radical having 6 to 22 carbon atoms, R¹² is hydrogen or an alkyl group containing 1 to 4 carbon atoms, R¹³ is an alkyl group containing 1 to 4 carbon atoms, q1 is a number of 1 to 6 and X is an alkali and/or alkaline earth metal or ammonium. Typical examples are the carboxymethylation products of hexylmethylamine, hexyldimethylamine, octyldimethylamine, decyldimethylamine, C_{12/14}-cocoalkyldimethylamine, myristyldimethylamine, cetyldimethylamine, stearyldimethylamine, stearylethylmethylamine, oleyldimethylamine, C_{16/18}-tallowalkyldimethylamine and their technical mixtures, and particularly dodecyl methylamine, dodecyl dimethylamine, dodecyl ethylmethylamine and technical mixtures thereof. The commercially available products include Dehyton^{®} AB (Cognis Deutschland GmbH & Co., KG)

Alkylamido betaines (a7) represent carboxyalkylation products of amidoamines which correspond to formula **(VIII):** 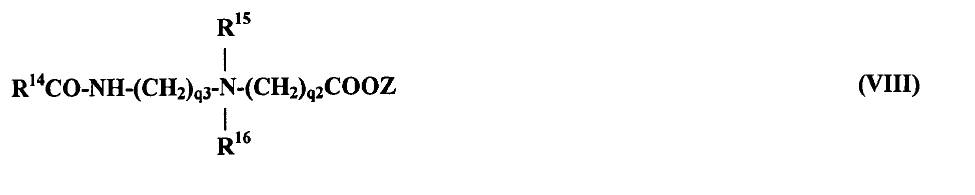 in which R¹⁴CO is an aliphatic acyl radical having 6 to 22 carbon atoms and 0 or 1 to 3 double bonds, R¹⁵ is hydrogen or an alkyl radical having 1 to 4 carbon atoms, R¹⁶ is an alkyl radical having 1 to 4 carbon atoms, q2 is a number from 1 to 6, q3 is a number from 1 to 3 and Z is an alkali and/or alkaline earth metal or ammonium. Typical examples are reaction products of fatty acids having 6 to 22 carbon atoms, like for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine und N,N-diethylaminopropylamine, which are condensed with sodium chloroacetate. The commercially available products include Dehyton^{®} K and Dehyton^{®} PK (Cognis Deutschland GmbH & Co., KG) as well as Tego^{®}Betaine (Goldschmidt).

### Dyes

Examples for suitable dyes are any of the substances suitable and approved for cosmetic purposes as listed, for example, in the publication **"**Kosmetische Farbemittel" of the Farbstoffkommission der Deutschen Forschungsgemeinschaft, Verlag Chemie, Weinheim, 1984, pages 81 to 106. Examples include cochineal red A (C.I. 16255), patent blue V (C.I. 42051), blue (C.I. 77077), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye.

### Micro-encapsulated dyes

In order to achieve the desired colouring effect by breaking the capsules during liquid soap and hand washing on the one side and reducing the amount of micro-capsules in the composition on the other side, another preferred embodiment of the present invention is directed to the use of micro-capsules showing a high content of dyes, particularly of micro-capsules containing 5 to 95 % b.w. and preferably 20 to 50 % b.w. of cosmetically acceptable dyes.

"Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as micro-particles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semi-synthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semi-synthetic membrane materials are, inter alia, chemically modified celluloses, more particularly, cellulose esters and ethers, for example cellulose acetate, ethyl cellulose, hydroxypropyl cellulose, hydroxypropyl methyl cellulose and carboxymethyl cellulose, and starch derivatives, more particularly, starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets): Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The active principles are released from the microcapsules by mechanical, thermal, chemical or enzymatic destruction of the membrane, normally during the use of the preparations containing the microcapsules. From the state of the art is known also a huge number of different processes for the encapsulation of active principles: WO 99/043426**;** WO 01/001928**;** WO 01/001929**;** WO 01/066240**;** WO 01/066241**;** WO 01/098578**;** WO 02/0178859**;** WO 02/0178868**;** WO 02/076607**;** WO 02/076606**;** WO 02/077359**;** WO 02/077360**;** WO 03/022419**;** WO 03/093571**;** WO 03/092664**;** WO 03/092880**;** WO 04/091555**;** WO 04/106621**;** EP 1064911 B1**;** EP 1064912 B1**;** EP 1077060 B1**;** EP 1101527 B1**;** EP 1223243 B1**;** EP 1243318 B1**;** EP 1243320 B1**;** EP 1243323 B1**;** EP 1243324 B1**;** EP 1254983 B1**;** EP 1121542 B1 all filed on behalf of Henkel KGaA, Primacare S.A. or Cognis Iberia, S.L. and herewith incorporated by reference.

Despite the fact that the state of the art cites a huge range of possibilities for the encapsulation of actives, methods according to which a shell is obtained by coacervation, precipitation or polycondensation of anionic and cationic polymers has been quite suitable for the formation of stable capsules. It should be noticed, however, that not all types of micro-capsules fulfil the requirements to solve the underlying problem of the invention in the same way. As a matter of fact, those micro-capsules are preferred, which are sufficiently stable in the presence of surfactants and exhibit also a high stability against any undesired migration of the dye through the shell into the care composition. In addition, although they should break during use, they must be sufficiently stable against mechanical treatment so that they do not break immediately, but after a defined period of time in order to signal that the hands are clean.

Taking this into account, a first type of preferred capsules and a respective process for the encapsulation of active principles according to the present invention is characterised in that it comprises the steps of
(a) preparing a matrix from gel formers, cationic polymers and active principles;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged. In another embodiment, the microcapsules according to the present invention can be prepared according to the following two processes:
(a3) preparing a matrix from gel formers, cationic polymers and active principles; and
(b3) dropping said dispersed matrix into aqueous solutions of anionic polymers or iono-tropically gellyfying anionic agents.
or
(a4) preparing a matrix from gel formers, anionic polymers and active principles; and
(b4) dropping said dispersed matrix into aqueous solutions of cationic molecules or iono-tropically gellifying cationic agents.

### Gel formers

In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3- and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1% by weight aqueous solution still form gels that do not melt below 80° C and solidify again above 40° C. Examples from the group of thermogelling proteins are the various gelatines.

### Anionic polymers

Salts of alginic acid are preferred for this purpose. The alginic acid is a mixture of carboxyl-containing polysaccharides with the following idealized monomer unit:

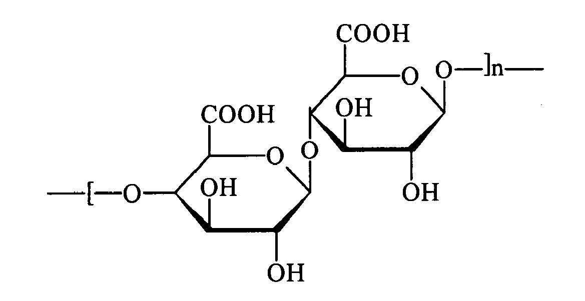

The average molecular weight of the alginic acid or the alginates is in the range from 150,000 to 250,000. The salts of alginic acid and complete and partial neutralization products thereof are in particular understood to be alkali metal salts, preferably sodium alginate ("algin") and the ammonium and alkaline earth metal salts. Mixed alginates, for example so-dium/magnesium or sodium/calcium alginates, are particularly preferred. In an alternative embodiment of the invention, however, carboxymethylcelluloses or anionic chitosan derivatives, for example the carboxylation and, above all, succinylation products are also suitable for this purpose.

### Cationic polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Preferred cationic polymers are cationic chitin derivatives such as, for example chitosan, optionally in microcrystalline distribution. Chitosans are biopolymers which belong to the group of hydrocolloids. Chemically, they are partly de-acetylated chitins differing in their molecular weights which contain the following - idealized - monomer unit:

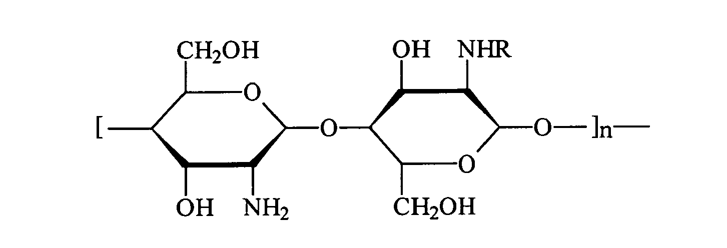

In contrast to most hydrocolloids, which are negatively charged at biological pH values, chitosans are cationic biopolymers under these conditions. The positively charged chitosans are capable of interacting with oppositely charged surfaces and are therefore used in cosmetic hair- care and body-care products and pharmaceutical preparations. Chitosans are produced from chitin, preferably from the shell residues of crustaceans which are available in large quantities as inexpensive raw materials. In a process described for the first time by Hackmann et al., the chitin is normally first de-proteinized by addition of bases, de-mineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1 % by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates.

In a preferred embodiment of the present invention anionic polymers of the alginate and carboxymethylcellulose type are reacted with cationic polymers of the chitosan type.

In another preferred embodiment of the invention, an aqueous solution of the gel former of 1 to 10, and preferably 2 to 5 % by weight, preferably agar agar, is normally prepared and heated under reflux. A second aqueous solution containing the cationic polymer, preferably chitosan, in quantities of 0.1 to 2, and preferably 0.25 to 0.5 % by weight and the active principle - the dye - in quantities of 0.1 to 25 and preferably 10 to 20 % by weight, is added in the boiling heat, preferably at 80 to 100°C; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilizers to the matrix. After its preparation from gel former, cationic polymer and active principle, the matrix is optionally very finely dispersed in an oil phase with intensive shearing in order to produce small particles in the subsequent encapsulation process. It has proved to be particularly advantageous in this regard to heat the matrix to temperatures in the range from 40 to 60° C while the oil phase is cooled to 10 to 20° C. The actual encapsulation, i.e. formation of the membrane by contacting the cationic polymer in the matrix with the anionic polymers, takes place in the third step. To this end, it is advisable to wash the matrix - dispersed in the oil phase - with an aqueous solution of the anionic polymer of approx. 0.1 to 3 and preferably 0.25 to 0.5% by weight, preferably the alginate, at a temperature in the range from 40 to 100 and preferably 50 to 60° C and, at the same time, to remove the oil phase if present. The resulting aqueous preparations generally have a microcapsule content of 1 to 20% by weight. In some cases, it can be of advantage for the solution of the polymers to contain other ingredients, for example emulsifiers or preservatives. After filtration, microcapsules with an average diameter of preferably 1 to 3 mm are obtained. It is advisable to sieve the capsules to ensure a uniform size distribution. The microcapsules thus obtained may have any shape within production-related limits, but are preferably substantially spherical. Their average diameter can be controlled by the speed of agitation during the incorporation into the cosmetic oil phase and lies between 0.0001 and 5, preferably 0.001 and 1 mm.

### Mild cleansing compositions

Preferably said mild cleansing composition comprise
(a) 5 to 50, preferably 10 to 25 % b.w. anionic and/or amphoteric surfactants
(b) 0.01 to 1, preferably 0.2 to 0.5 % b.w. dyes and
(c) 1 to 50, preferably 2 to 10 % b.w. encapsulated dyes, showing a colour different from the dyes of component (b)
on condition that the amounts add with water and optionally other typical additives to 100 % b.w.

### Industrial application

Another object of the present invention concerns the use of mild cleansing compositions comprising
(a) Anionic, non-ionic and/or amphoteric surfactants
(b) Dyes and
(c) Encapsulated dyes, showing a colour different from the dyes of component (b)
for manufacturing personal care compositions, manual dish washing compositions or light duty detergents, in particular for making liquid soaps, hand washing pastes or tooth pastes.

### Cosmetic compositions

The preparations according to the invention may in addition contain co-surfactants, oil bodies, emulsifiers, superfatting agents, pearlising waxes, consistency factors, polymers, silicone compounds, waxes, stabilizers, primary and secondary sun protection agents, antidandruff agents, biogenic agents, film formers, swelling agents, hydrotropes, preservatives, solubilizers, complexing agents, reducing agents, alkalising agents, perfume oils, and the like.

### Co-Surfactants

Other preferred co-surfactants are anionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### Polyglycerol esters

Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan® PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid for example, and C₁₂-₂₂ dicarboxylic acids, such as azelaic acid or sebacic acid for example.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquae^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**)**.

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
- 3-benzylidene camphor or 3-benzylidene norcamphor and derivatives thereof, for example 3-(4-methylbenzylidene)-camphor;
- 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
- esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
- esters of salicylic acid, preferably salicylic acid-2-ethylhexyl ester, salicylic acid-4-isopropylbenzyl ester, salicylic acid homomenthyl ester;
- derivatives of benzophenone, preferably 2-hydroxy-4-methoxybenzophenone, 2-hydroxy-4-methoxy-4'-methylbenzophenone, 2,2'-dihydroxy-4-methoxybenzophenone;
- esters of benzalmalonic acid, preferably 4-methoxybenzalmalonic acid di-2-ethylhexyl ester;
- triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb^{®} HEB);
- propane-1,3-diones such as, for example, 1-(4-tert.butylphenyl)-3-(4'-methoxyphenyl)-propane-1,3 -dione;
- ketotricyclo(5.2.1.0)decane derivatives.

Suitable water-soluble substances are
- 2-phenylbenzimidazole-5-sulfonic acid and alkali metal, alkaline earth metal, ammonium, alkylammonium, alkanolammonium and glucammonium salts thereof;
- sulfonic acid derivatives of benzophenones, preferably 2-hydroxy-4-methoxybenzophenone-5-sulfonic acid and salts thereof;
- sulfonic acid derivatives of 3-benzylidene camphor such as, for example, 4-(2-oxo-3-bomylidenemethyl)-benzene sulfonic acid and 2-methyl-5-(2-oxo-3-bomylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}, in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propyl-thiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihy-droguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Anti-microbial agents

Suitable anti-microbial agents are, in principle, all substances effective against Gram-positive bacteria, such as, for example, 4- hydroxybenzoic acid and its salts and esters, N-(4-chlorophenyl)-N'-(3,4- dichlorophenyl)urea, 2,4,4'-trichloro-2'-hydroxy-diphenyl ether (triclosan), 4-chloro-3,5-dimethyl-phenol, 2,2'-methylenebis(6-bromo-4- chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n- decylsalicylamide.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Odour absorbers

Suitable odour absorbers are substances which are able to absorb and largely retain odour-forming compounds. They lower the partial pressure of the individual components, thus also reducing their rate of diffusion. It is important that perfumes must remain unimpaired in this process. Odour absorbers are not effective against bacteria. They comprise, for example, as main constituent, a complex zinc salt of ricinoleic acid or specific, largely odour-neutral fragrances which are known to the person skilled in the art as "fixatives", such as, for example, extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal products, such as, for example, civet and castoreum. Typical synthetic fragrance compounds are products of the ester, ether, aldehyde, ketone, alcohol, and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linaool, phenylethyl alcohol and terpineol, and the hydrocarbons include mainly the terpenes and balsams. Preference is, however, given to using mixtures of different fragrances which together produce a pleasing fragrance note. Essential oils of relatively low volatility, which are mostly used as aroma components, are also suitable as perfume oils, e.g. sage oil, camomile oil, oil of cloves, melissa oil, mint oil, cinnamon leaf oil, linden flower oil, juniperberry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β- damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

### Antiperspirant active ingredients

Suitable astringent antiperspirant active ingredients are primarily salts of aluminium, zirconium or of zinc. Such suitable antihydrotic active ingredients are, for example, aluminium chloride, aluminium chlorohydrate, aluminium dichlorohydrate, aluminium sesquichlorohydrate and complex compounds thereof, e.g. with 1,2- propylene glycol, aluminium hydroxyallantoinate, aluminium chloride tartrate, aluminium zirconium trichlorohydrate, aluminium zirconium tetrachlorohydrate, aluminium zirconium pentachlorohydrate and complex compounds thereof, e.g. with amino acids, such as glycine.

### Film formers

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

### Antidandruff agents

Suitable antidandruff agents are Pirocton Olamin (1-hydroxy-4-methyl-6-(2,4,4-trimethylpentyl)-2-(1H)-pyridinone monoethanolamine salt), Baypival^{®} (Climbazole), Ketoconazol® (4-acetyl-1-{4-[2-(2,4-dichlorophenyl) r-2-(1H-imidazol-1-ylmethyl)-1,3-dioxylan-c-4-yl-methoxyphenyl}-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

The total percentage content of auxiliaries and additives may be from 1 to 50% by weight and is preferably from 5 to 40% by weight, based on the particular composition. The compositions may be produced by standard hot or cold processes.

### Examples

### Example 1

### Preparation of Dye Primasponge ^{®} Blue Capsules

A first pre-mixture "A" was prepared by dissolving 1 g agar-agar in 43 ml of boiling water. For pre-mixture "B" 0.5 g Phenonip^{®} was dissolved in 15 ml of a 2 % b.w. aqueous solution of sodium alginate. A third pre-mixture "C" was obtained by dispersing 20 g of a blue dye (CI 77007) in 30 g of glycerol. Finally, a pre-mixture "D" was prepared by mixing 5 g shea butter (Cetiol^{®} SB-45) with 0.5 g of a non-ionic emulsifier (Tween^{®}-20). Subsequently, pre-mixtures B, C and D were added stepwise to pre-mixture A. The mixture thus obtained was kept at about 70 °C, that means a temperature above the gelification point of the agar, and was slowly dispersed under agitation into 250 ml of a cosmetic oil (Cetiol^{®} 868) having a temperature of about 20 °C. When dropped into the cold oil, the droplets gelled and blue spheres were formed, their diameter being controlled by the speed of agitation. Afterwards, the capsules were removed by filtration, washed with an aqueous solution comprising 1 % Tween-20 and 0.1 b.w. % of chitosan glycolate. Finally, the capsules were dispersed in an external phase comprising 10 % propylene glycol, 0.4 % b.w. Phenonip, and 0.4 % b.w. phenoxy ethanol.

### Example 2

### Preparation of a liquid soap with encapsulated dyes

**Table 1**

| **Liquid soap** | | | |
|---|---|---|---|
| **Phase** | **Components** | **INCI** | **% [b.w.]** |
| I | Water | | 30.0 |
| | Carbopol^{®} Aqua SF 1 | Acrylates copolymer | 8.0 |
| | NaOH dil. 10 % | | appr. 3.4 |
| II | Water | | up to 100 |
| | Glycerine | Glycerol | 1.0 |
| | Hexylene Glycol | Hexylene glycol | 1.0 |
| | Plantapon^{®} 611C | Sodium laureth sulfate (and) Cocamidopropyl Betaine (and) Coco Glucoside | 20.0 |
| | Yellow Dye | Quinoline yellow (C.I. 47005) | 0.2 |
| | Irgasan^{®} DP 300 | Triclosan | 0.3 |
| III | Cetiol^{®} LDO | Dicaprylyl Ether (and) Lauryl Alcohol | 0.2 |
| IV | Dye Primasponge^{®} Blue | Blue dye, encapsulated | 4.0 |

The ingredients of Phase I were mixed until transparency was reached and the pH value adjusted to 6.7 to 7.2. The ingredients of Phase II ware stirred until homogeneity was reached and also adjusted to a pH of 6.7 - 7.2. Subsequently, Phase II was given over Phase I avoiding any incorporation of air. Then the oil bodies (Phase III) were added to the composition, while the *Primasponges*^{®} were added as the last component under gentle stirring. The paste showed a viscosity according to Brookfield (RVT, spd 3,10 rpm) of 2,700 mPas.

## Claims

1. Mild cleansing compositions comprising
(a) Anionic, non-ionic and/or amphoteric surfactants
(b) Dyes and
(c) Encapsulated dyes, showing a colour different from the dyes of component (b).

2. Compositions according to Claim 1, **characterised in that** they represent personal care compositions, manual dish washing compositions or light duty detergents.

3. Compositions according to Claim 2, **characterised in that** they represent liquid soaps, hand washing pastes or tooth pastes.

4. Compositions according to any of the Claims 1 to 3, **characterised in that** they represent aqueous pastes showing a viscosity according to Brookfield (RVT, spindle 3, 10 rpm) of 300 to 30,000 mPas.

5. Compositions according to any of the Claims 1 to 4, **characterised in that** they comprise mild anionic and/or amphoteric surfactants selected from the group consisting of N-acyl amino acids, alk(en)yl oligoglycoside carboxylates, alk(en)yl polyalkylene glycol ether citrates, alk(en)yl oiloglycosides, alkyl betains and alkylamido betaines.

6. Compositions according to any of the Claims 1 to 5, **characterised in that** they comprise cosmetically acceptable dyes selected from the group consisting of cochineal red A (C.I. 16255), patent blue V (C.I. 42051), blue (C.I. 77077), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000).

7. Compositions according to any of Claims 1 to 6, **characterised in that** they comprise micro-encapsulated dyes, said micro-capsules obtained from coacervation, precipitation or polycondensation of anionic and cationic polymers.

8. Compositions according to any of Claims 1 to 6, **characterised in that** they comprise micro-encapsulated dyes, said micro-capsules obtained from reaction of alginates or carboxymethylcelluloses with chitosan.

9. Compositions according to any of the Claims 1 to 7, **characterised in that** they comprise
(a) 5 to 50 % b.w. anionic and/or amphoteric surfactants
(b) 0.01 to 1 % b.w. dyes and
(c) 1 to 50 % b.w. encapsulated dyes, showing a colour different from the dyes of component (b)
on condition that the amounts add with water and optionally other typical additives to 100 % b.w.

10. Use of cleansing compositions comprising
(a) anionic and/or amphoteric surfactants
(b) Dyes and
(c) Encapsulated dyes, showing a colour different from the dyes of component (b)
for manufacturing personal care compositions, manual dish washing compositions or light duty detergents.

11. Use according to Claim 10, **characterised in that** said compositions represent liquid soaps, hand washing pastes or tooth pastes.
